Europäisches Patentamt
European Patent Office
Office européen des brevets

(11) Publication number: 0 221 541 A2

# EUROPEAN PATENT APPLICATION

(21) Application number: 86115285.8

(22) Date of filing: 04.11.86

(51) Int.Cl.[4]: C 07 D 401/04, A 61 K 31/47

(30) Priority: 05.11.85 JP 247490/85

(43) Date of publication of application: 13.05.87 Bulletin 87/20

(84) Designated Contracting States: BE CH DE FR GB IT LI NL SE

(71) Applicant: KYORIN PHARMACEUTICAL CO., LTD.
No. 5, Kanda Surugadai 2-chome
Chiyoda-ku Tokyo(JP)

(72) Inventor: Masuzawa, Kuniyoshi
No. 5-71, Nishi-cho
Koga-shi Ibaragi-ken(JP)

(72) Inventor: Suzue, Seigo
No. 13-4 Aoba 4-chome
Kuki-shi Saitama-ken(JP)

(72) Inventor: Hirai, Keiji
No. 1-2-512, Aoba 1-chome
Kuki-shi Saitama-ken(JP)

(72) Inventor: Ishizaki, Takayoshi
No. 9-6, Sakurada 4-chome Washimiya-machi
Kitakatsushika-gun Saitama-ken(JP)

(74) Representative: Patentanwälte TER MEER - MÜLLER - STEINMEISTER
Mauerkircherstrasse 45
D-8000 München 80(DE)

## (54) Quinolonecarboxylic acid derivatives and their preparation.

(57) Quinolonecarboxylic acid derivatives of the following formula,

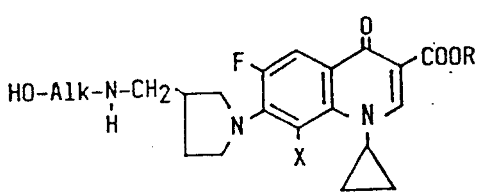

wherein R is hydrogen atom or lower alkyl group, Alk is lower alkylene group and X is hydrogen atom or halogen atom; the hydrates and pharmaceutically acceptable salts thereof are useful as antibacterial agent.

EP 0 221 541 A2

TER MEER-MÜLLER-STEINMEISTER
PATENTANWÄLTE - EUROPEAN PATENT ATTORNEYS

Dipl.-Chem. Dr. N. ter Meer
Dipl. Ing. F. E. Müller
Mauerkircherstrasse 45
D-8000 MÜNCHEN 80

Dipl. Ing. H. Steinmeister
Artur-Ladebeck-Strasse 51
D-4800 BIELEFELD 1

tM/cb
K-2377-03

November 4, 1986

KYORIN PHARMACEUTICAL CO., LTD.
No. 5, Kanda Surugadai 2-chome
Chiyoda-ku, Tokyo, Japan

# Quinolonecarboxylic acid derivatives and their preparation

Priority: November 5, 1985, Japan, No. 60-247490 (P)

Detailed description of the invention:

This invention is concerned with certain novel useful quinolonecarboxylic acid derivatives of the formula (I), with a process for their preparation and with compositions containing them.

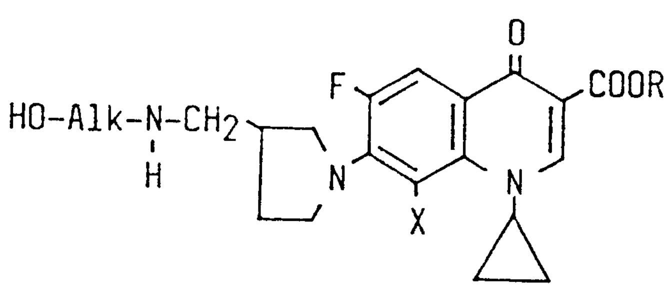

(I)

In the formula (I), R is hydrogen atom or lower alkyl group, Alk is lower alkylene group and X is hydrogen atom or halogen atom.

The term "lower alkyl group", as used herein, means alkyl radicals having from one to five carbon atoms such as methyl, ethyl, isopropyl and t-butyl group.

The term "lower alkylene group", as used herein, means alkylene radicals having from one to five carbon atoms, of which can be substituted by above mentioned lower alkyl group, as illustrated by ethylene, propylene, 2-methylpropylene and pentamethylene.

The term "halogen atom", as used herein, means fluorine, chlorine, bromine and iodine, especially fluorine and chlorine.

Since nalidixic acid which has been employed for treatment of urinary tract infections by gram-negative bacteria, was introduced in 1963, intensive work has been carried out on the further development of quinolonecarboxylic acid analogue.

Thus, recently a remarkable antibacterial activity against not only gram-negative bacteria but also gram-positive bacteria occurs for some compounds (e.g. norfloxacin). However their activity against gram-positive bacteria is fairly less than that against gram-negative bacteria.

Just recently, the drugs (e.g. CI-934) which have relatively strong activity against gram-positive bacteria have been developed, but shown to possess weaker activity against gram-negative bacteria than that of the prior compounds (e.g. norfloxacin, ciprofloxacin).

As a result of the investigation, the present inventors have now unexpectedly found that new derivatives of quinolonecarboxylic acid represented by the formula (I) have excitingly potential activity against gram-negative bacteria in comparison with that of any prior analogue and therefore are superior to commercial preparation and investigational drugs in the in vitro

and *in vivo* antibacterial activity against gram-negative and gram-positive bacteria.

Furthermore, the compounds of this invention possess excellent antibacterial activity not only against aerobic bacteria but also against anaerobic bacteria and mycoplasmas.

The present compounds are well absorbed and distributed into the tissue when administered orally in animals.

The present compounds, therefore, are active at low doses against both gram-positive and gram-negative bacteria and thus constitute valuable agents for the treatment of infectious human, animal or plant diseases.

In following, explanation is made about the preparation process for the compound of the invention.

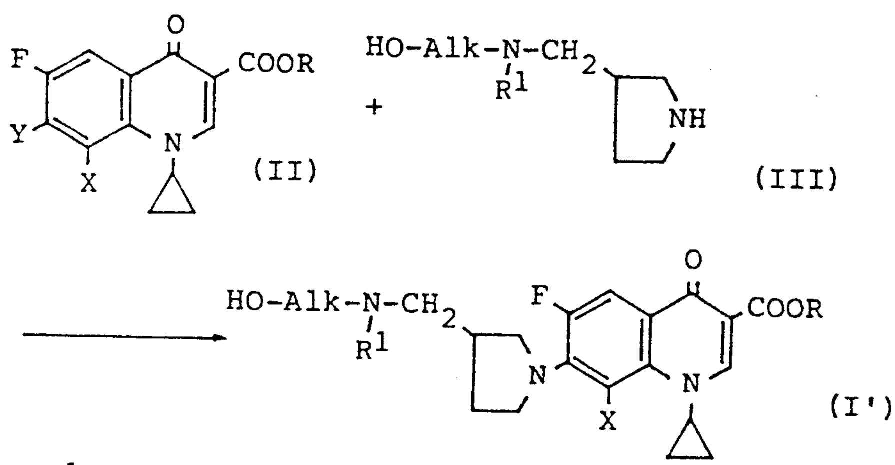

(II) (III)

(I')

wherein $R^1$ is hydrogen atom or protective group, Y is halogen atom and Alk, R and X have the above-stated meanings.

Namely, by allowing compounds represented by the formula (II) to react with amines presented by the formula (III), compounds represented by the formula (I') are synthesized. However, in the case of compounds wherein $R^1$ is protective group in the formula (III), e.g., $R^1$ is alkoxycarbonyl group, for example,

methoxy, ethoxy, t-butoxy-, benzyloxycarbonyl group and the acyl group, e.g., formyl, acetyl, propionyl, benzoyl group and the like, the protective group is removed from the reactant represented by the formula (I') according to the usual method to give the compounds of this invention, wherein $R^1$ is hydrogen atom. The reaction of compounds represented by the formula (II) with amines represented by the formula (III) preferably is carried out by heating the mixture in a solvent such as water, alcohols, acetonitrile, dimethylformamide (DMF), dimethyl sulfoxide (DMSO), hexamethylphosphoric triamide, pyridine, picoline and the like or in the absence of the solvent. The reaction temperature is selected appropriately in a range of room temperature to 200 °C, preferably room temperature to 160 °C. In more details, it is preferable to allow compounds represented by the formula (II) to react with 1 to 5 times mole of compounds represented by the formula (III) for 1 to several hours at room temperature to 160 °C in 2 to 10 times volume of aforementioned solvents. At this time, the use of deacidifying agents such as triethylamine, diazabicyclo bases and potassium carbonate is also desirable. Moreover, compounds (I') wherein R is a lower alkyl group can be hydrolyzed to give the carboxylic acids according to the usual method. Such hydrolysis can be carried out easily with alkalies such as potassium hydroxide or acids such as sulfuric acid at room temperature to boiling point of solvents in water, mixed liquor of water with alcohols, mixed liquor of water with acetic acid, and so on.

Furthermore, the compounds of the formula (I) can be converted, if desired, to the pharmaceutically acceptable ammonium salts or carboxylic acid metal salts by treatment with acid or alkali. The acid may be organic or inorganic acids such as, for example, hydrochloric acid, sulfuric acid, phosphoric acid, acetic acid, methanesulfonic acid, oxalic acid and lactic acid. The carboxylic acid metal salts may be, for example, sodium, potassium, magnesium, calcium, aluminum, cerium, chromium, cobalt, copper, iron, zinc, platinum and silver salts.

The compound of the formula (I), hydrates and salts thereof may be used as medicines in the conventional form of pharmaceutical preparations, which may be, for example, tablets, capsules, powder, ointments, suppositories, injections or eye drops, suitable for peroral, parenteral, enteral or local administration.

The following examples will further illustrate the invention without, however, limiting it thereto.

Example 1. 1-Cyclopropyl-6,8-difluoro-1,4-dihydro-7-[3-(2-hydroxyethylaminomethyl)-1-pyrrolidinyl]-4-oxo-3-quinolinecarboxylic acid

A suspension of 1-cyclopropyl-6,7,8-trifluoro-1,4-dihydro-4-oxo-3-quinolinecarboxylic acid (600 mg), 2-(3-pyrrolidinylmethylamino)ethanol (340 mg) and 1,8-diazabicyclo[5,4,0]-7-undecene (DBU, 330 mg) in absolute acetonitrile (5 ml) was refluxed for 1.5 hours and further stirred at room temperature for 4 hours. The resulting precipitate was collected by filtration and washed with ethanol to give the title compound (800 mg), mp 265-267.5 °C (decompd.).

Analysis (%) for $C_{20}H_{23}F_2N_3O_4$, Calcd. (Found): C, 58.96

(58.80); H, 5.69 (5.74); N, 10.31 (10.53).

Example 2. 1-Cyclopropyl-6-fluoro-1,4-dihydro-7-[3-(2-hydroxyethylaminomethyl)-1-pyrrolidinyl]-4-oxo-3-quinolinecarboxylic acid hydrochloride

A suspension of 7-chloro-1-cyclopropyl-6-fluoro-1,4-dihydro-4-oxo-3-quinolinecarboxylic acid (500 mg) and 2-(3-pyrrolidinyl-methylamino)ethanol (780 mg) in ß-picoline (5 ml) was refluxed for 80 minutes and further stirred at room temperature for 4 hours. Then to the reacting mixture was added concentrated aqueous ammonia (10 ml) and concentrated. The residue was dissolved in methanol-concentrated hydrochloric acid (2:1, 83 ml) and concentrated. The residue was recrystallized from water to give the title compound (520 mg), mp 266-282 °C (decompd.).

Analysis (%) for $C_{20}H_{24}FN_3O_4 \cdot HCl \cdot 1/2\ H_2O$, Calcd. (Found): C, 55.24 (55.36); H, 6.03 (5.92); N, 9.66 (9.81).

Example 3. 8-Chloro-1-cyclopropyl-6-fluoro-1,4-dihydro-7-[3-(2-hydroxyethylaminomethyl)-1-pyrrolidinyl]-4-oxo-3-quinolinecarboxylic acid

A suspension of 8-chloro-1-cyclopropyl-6,7-difluoro-1,4-dihydro-4-oxo-3-quinolinecarboxylic acid (500 mg), 2-(3-pyrrolidinylmethylamino)ethanol (270 mg) and DBU (260 mg) in absolute acetonitrile (5 ml) was refluxed for 2 hours and further stirred at room temperature for 4 hours. Then the resulting precipitate was collected by filtration and washed with ether to give the title compound (590 mg), mp 240-243 °C (decompd.).

Analysis (%) for $C_{20}H_{23}ClFN_3O_4$, Calcd. (Found): C, 56.67 (56.32); H, 5.47 (5.51); N, 9.91 (9.95).

Experiment 1. Antibacterial spectrum

Minimal inhibitory concentrations (MICs) were determined in accordance with the method recommended by Japan Society of Chemotherapy. The results are shown in Table 1.

Table 1-a In vitro antibacterial activity (aerobic bacteria)

| Organism ($10^6$ cells/ml) | Gram | MIC (μg/ml) | | | |
|---|---|---|---|---|---|
| | | Exp. 1 | Exp. 2 | Exp. 3 | CPFX |
| Bacillus subtilis PCI 219 | + | 0.0125 | ≤0.05 | 0.0125 | 0.05 |
| Staphylococcus aureus 209 P | + | 0.05 | 0.20 | 0.0125 | 0.20 |
| S. aureus Smith | + | 0.05 | 0.39 | 0.0125 | 0.39 |
| S. aureus IID 670 (Terajima) | + | 0.05 | 0.20 | 0.025 | 0.20 |
| S. epidermidis IID 866 | + | 0.025 | 0.10 | 0.0125 | 0.20 |
| E. faecalis IID 682 | + | 0.10 | 0.78 | 0.10 | 0.78 |
| Streptococcus pneumoniae IID 552 | + | 0.05 | 0.10 | 0.0125 | 0.78 |
| S. pyogenes IID 689 (S-8) | + | 0.025 | 0.10 | 0.0125 | 0.78 |
| S. pyogenes IID 692 | + | 0.05 | 0.10 | 0.0125 | 0.78 |
| Escherichia coli NIHJ JC-2 | - | 0.025 | 0.10 | 0.0125 | ≤0.0063 |
| E. coli ATCC 10536 | - | 0.05 | 0.10 | 0.025 | 0.0125 |
| E. coli ML 4707 | - | 0.05 | 0.20 | 0.05 | 0.0125 |
| Proteus vulgaris IFO 3167 | - | 0.05 | 0.20 | 0.025 | 0.0125 |
| P. mirabilis IID 994 | - | 0.05 | 0.39 | 0.05 | 0.0125 |
| Morganella morganii IID 602 | - | 0.39 | 3.13 | 0.39 | 0.025 |
| Enterobacter cloacae IID 977 | - | 0.20 | 1.56 | 0.10 | 0.025 |
| Citrobacter freundii IID 976 | - | 0.05 | 0.39 | 0.05 | 0.0125 |
| Klebsiella pneumoniae KY(GN)6445 | - | 0.05 | 0.39 | 0.05 | 0.0125 |
| K. pneumoniae 1-220S | - | 0.20 | 0.78 | 0.10 | 0.05 |
| Salmonella enteritidis IID 604 | - | 0.10 | 0.39 | 0.10 | 0.0125 |
| Shigella sonnei IID 969 | - | 0.05 | 0.10 | 0.05 | 0.0125 |
| Yersinia enterocolitica IID 981 | - | 0.20 | 0.78 | 0.10 | 0.05 |
| Serratia marcescens IID 618 | - | 0.39 | 1.56 | 0.20 | 0.05 |
| S. marcescens GN 7577 | - | 3.13 | 25 | 1.56 | 0.39 |
| Pseudomonas aeruginosa V-1 | - | 0.78 | 1.56 | 0.20 | 0.05 |
| P. aeruginosa IFO 12689 | - | 1.56 | 6.25 | 1.56 | 0.20 |
| P. aeruginosa IID 1210 | - | 1.56 | 6.25 | 1.56 | 0.20 |
| P. cepacia GIFU 518 | - | - | - | 1.56 | 0.39 |
| P. cepacia KY 84 | - | 3.13 | - | - | - |
| P. maltophilia GIFU 2491 | - | - | - | 0.20 | 0.39 |
| Acinetobacter anitratus IID 876 | - | 0.20 | 3.13 | 0.10 | 0.10 |
| Alcaligenes faecalis 0114002 | - | 0.78 | 6.25 | 0.39 | 0.20 |

Table 1-b In vitro antibacterial activity (anaerobic bacteria)

| Organism ($10^6$ cells/ml) | Gram | MIC (μg/ml) | | | |
|---|---|---|---|---|---|
| | | Exp. 1 | Exp. 2 | Exp. 3 | CPFX |
| Bacteroides fragilis GM 7000 | - | 1.56 | 100 | 0.39 | 6.25 |
| B. fragilis 0558 | - | 0.78 | 50 | 0.20 | 3.13 |
| B. fragilis 25285 | - | 1.56 | 50 | 0.20 | 6.25 |
| B. distasonis 8503 | - | - | - | 0.78 | 6.25 |
| B. thetaiotaomicron (0661) | - | 3.13 | >100 | 1.56 | 12.5 |
| Fusobacterium necrophorum S-45 | - | 0.20 | 0.78 | 0.10 | 1.56 |
| F. varium KYA 8501 | - | 0.78 | 25 | 0.39 | 25 |
| Eubacterium lentum GAI 5242 | + | 0.78 | 3.13 | 0.20 | 0.78 |
| Propionibacterium acens 11828 | + | 0.78 | 3.13 | 0.78 | 12.5 |
| Peptococcus magnus KY 017 | + | ≤0.05 | 0.20 | 0.05 | 0.39 |
| Clostridium difficile I-E | + | 0.78 | 25 | 0.20 | 12.5 |
| C. perfringens KYA 13123 | + | 0.20 | 1.56 | 0.05 | 0.39 |
| C. ramosum | + | 0.78 | 25 | 0.39 | 12.5 |
| Peptostreptococcus anaerobius KYA 27337 | + | ≤0.05 | 0.78 | 0.025 | 1.56 |
| Pst. micros UPI 5464-1 | + | 0.39 | 3.13 | 0.10 | 0.20 |
| Veillonella parvula KYA 10790 | - | 0.39 | 3.13 | 0.10 | 0.20 |

CPFX : Ciprofloxacin

What is claimed is :

1. A compound of the formula (I);

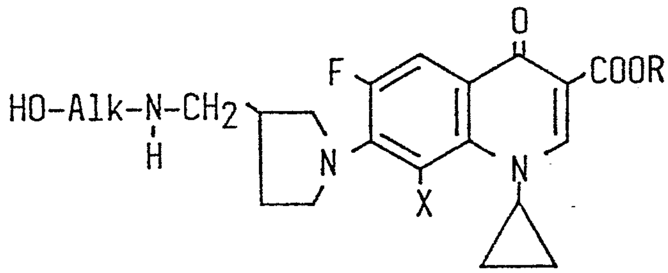

(I)

wherein R is hydrogen atom or lower alkyl group, Alk is lower alkylene group and X is hydrogen atom or halogen atom; the hydrates or the pharmaceutically acceptable acid addition or alkali salts thereof.

2. A process for the preparation of a compound of the formula (I) specified in claim 1, which comprising a compound of the formula (II);

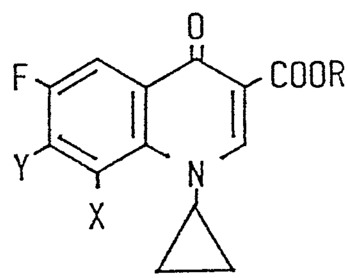

(II)

wherein R is hydrogen atom or lower alkyl group, X is hydrogen atom or halogen atom and Y is halogen atom; with a pyrrolidine derivative of the formula (III);

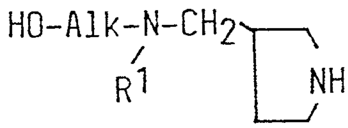

(III)

wherein Alk is lower alkylene group and $R^1$ is hydrogen atom or protecting group of amino group; however in which, when $R^1$ is

protecting group of amino group, the condensed product is followed by removal of the protecting group to amino derivative and when R is lower alkyl group, the condensed product is followed by hydrolizing to the carboxylic acid derivative.

3. An antibacterial pharmaceutical composition comprising at least one compound according to claim 1 and an inert pharmaceutically acceptable carrier.